# EUROPEAN PATENT SPECIFICATION

(11) **EP 2 956 539 B1**
(45) Date of publication and mention of the grant of the patent: **02.10.2019**
(21) Application number: 14751359.2
(22) Date of filing: 12.02.2014
(51) Int. Cl.: C12N 5/00, C12N 5/071

(54) **HIGH THROUGHPUT SCREENING OF AGENTS ON DOPAMINERGIC NEURONS**
SCREENING VON MITTELN AUF DOPAMINERGISCHEN NEURONEN MIT HOHEM DURCHSATZ
CRIBLAGE À HAUT DÉBIT POUR IDENTIFIER LES AGENTS AYANT UNE ACTION SUR LES NEURONES DOPAMINERGIQUES

(30) Priority: 13.02.2013 US 201361764031 P; 18.03.2013 US 201361802814 P
(43) Date of publication of application: 23.12.2015
(73) Proprietor: Hadasit Medical Research Services and Development Ltd., 9112001 Jerusalem (IL)
(72) Inventor: MORDECHAI DANIEL, Talya, 9972500 Doar-Na Shimshon (IL); WISER, Ofer, 9371422 Jerusalem (IL); REUBINOFF, Benjamin Eithan, 9988000 Doar-Na HaEla (IL)
(74) Representative: Vogel, Andreas
(86) International application number: PCT/IL2014/050149
(87) International publication number: WO 2014/125481

(56) References cited:
- WO-A1-2009/105481
- WO-A1-2012/116432
- US-A1- 2005 255 589
- US-A1- 2007 015 138
- RODRIGUEZ ET AL.: 'Fluorescent dopamine tracer resolves individual dopaminergic synapses and their activity in the brain.' PROC NAT ACAD SCI 15 January 2013, pages 870 - 875, XP055271226
- MAY ET AL.: 'In vitro expressed GPCR inserted in polymersome membranes for ligand-binding studies.' ANGEWANDTE CHEMIE INTERNATIONAL EDITION vol. 52, no. 2, 07 January 2013, pages 749 - 753, XP055271230
- KRIKS ET AL.: 'Floor plate-derived dopamine neurons from hESCs efficiently engraft in animal models of PD.' NATURE vol. 480, no. 7378, 06 November 2011, pages 547 - 551, XP055271235

## Description

### FIELD AND BACKGROUND OF THE INVENTION

The present invention, in some embodiments thereof, relates to screening of agent for an effect on dopaminergic neurons using fluorescent dopamine analogs.

Parkinson's disease is characterized by degeneration of dopaminergic (DA) neurons in the substantia nigra, resulting in movement abnormalities, rigidity and tremor. The symptoms may be transiently alleviated by L-DOPA and other drugs (i.e., dopamine receptor agonists); nevertheless, it has not been possible to stop the progression of the disease. Therefore, it is of great interest to identify novel compounds with neuroprotective and regenerative properties for the treatment of Parkinson's disease.

Screening of large libraries for compounds with potential therapeutic effects has not been performed in mammalian animal models due to high costs and time limitations. Hence, the development of a cell-based high-throughput screening (HTS) system to identify novel therapeutic compounds would be highly valuable.

Dopaminergic tracers have been developed and studied in single cell preparation, such as JHC1-64 (3, 4), a cocaine analog which binds specifically the dopamine transporter either on the cell surface or internalized transporters upon incubation. FFN511 is a fluorescent false neurotransmitter (U.S. Patent No. 8,337,941), which is transported by the VMAT into vesicles and is secreted in the dopaminergic terminals which can also label adrenergic and serotonergic neurons.

Dansyl D1 (dopamine labeled with dansyl molecule, Five-Photon Biochemical ™) has been suggested as an agent for high throughput screening and drug discovery.

Recently, other high throughput systems for the detection of dopaminergic neurons have been developed such as antagonist-conjugated quantum dots specific for the dopamine transporter DAT (2) as well as the fluorescent organic compound 4-(4-diethylaminostyryl)-N-methylpyridinium iodide (ASP+, 6) which is specific to all monoamines. Another system uses fluorescent cocaine analogs (3, 4) in which the fluorescent (rhodamine-, OR Green-, or Cy3) tags were extended from the tropane N-position of 2-beta-carbomethoxy-3beta-(3,4-dichlorophenyl) tropane using an ethylamino-linker.

Recently, a novel proprietary fluorescent indicator dye that mimics the neurotransmitters serotonin, norepinephrine, and dopamine, and is actively transported into the cells via the neurotransmitter transporters was developed into a commercially available assay kit - MDS Analytical Technologies, Molecular Devices (U.S. Patent Nos. 6,420,183, 7,063,952, 7,138,280 and European Patent No. 0,906,572).

Additional background art includes May et al., Angew. Chem. Int. Ed. 2013, 52, 749-753.

In references WO2009/105481 A1 and US2007/015138 A1 methods for identifying an agent or condition that modulates neurogenesis are disclosed.

### SUMMARY OF THE INVENTION

According to an aspect of some embodiments of the present invention there is provided a method of determining whether an agent is a neuroeffector, the method comprising:
(a) labeling dopaminergic neurons which are comprised in a mixed population of cells with a fluorescent dopamine analog;
(b) measuring a level of fluorescence in the mixed population of cells;
(c) exposing the mixed population of cells to the agent;
(d) remeasuring a level of fluorescence in the mixed population of cells, wherein a change in the level of fluorescence is indicative of the substance being a neuroeffector.

According to some embodiments of the invention, the mixed population of cells further comprises cells which express a dopamine receptor and do not express a dopamine transporter.

According to some embodiments of the invention, the dopaminergic neurons are generated by ex vivo differentiating pluripotent stem cells.

According to some embodiments of the invention, the pluripotent stem cells comprise embryonic stem (ES) cells.

According to some embodiments of the invention, the pluripotent stem cells comprise induced pluripotent stem (iPS) cells.

According to some embodiments of the invention, when the agent is a neurotoxin, the change in the level of fluorescence is a decrease.

According to some embodiments of the invention, when the agent is neurotrophic, the change in the level of fluorescence is an increase.

According to some embodiments of the invention, the labeling is effected in the presence of a dopamine receptor antagonist.

According to some embodiments of the invention, the fluorescent dopamine analog comprises Dansyl D1.

According to some embodiments of the invention, the dopamine receptor antagonist comprises sulpiride.

According to some embodiments of the invention, the method further comprises labeling the dopaminergic neurons with the fluorescent dopamine analog following step (c) and prior to step (d).

According to some embodiments of the invention, the agent does not bind to a dopamine receptor.

According to some embodiments of the invention, the agent is not transported through a dopamine transporter.

According to some embodiments of the invention, the ex vivo differentiating is effected by contacting the pluripotent stem cells in a medium comprising FGF8, Purmorphamine and CHIR99021.

According to an aspect of some embodiments of the present invention there is provided a method of determining whether an agent affects differentiation of a cell, the method comprising:
(a) inducing cells to differentiate into a mixed population of cells comprising dopaminergic neurons in the presence of the agent;
(b) labeling the dopaminergic neurons with a fluorescent dopamine analog;
(c) measuring a level of fluorescence in the mixed population of cells, wherein when the level is above a predetermined amount the agent is indicative as having a positive effect on dopaminergic differentiation.

According to some embodiments of the invention, the mixed population of cells further comprises cells which express a dopamine receptor and do not express a dopamine transporter.

According to some embodiments of the invention, the inducing cells to differentiate comprises inducing pluripotent stem cells to differentiate.

According to some embodiments of the invention, the pluripotent stem cells comprise embryonic stem (ES) cells.

According to some embodiments of the invention, the pluripotent stem cells comprise induced pluripotent stem (iPS) cells.

According to some embodiments of the invention, the labeling is effected in the presence of a dopamine receptor antagonist.

According to some embodiments of the invention, the fluorescent dopamine analog comprises Dansyl D1.

According to some embodiments of the invention, the dopamine receptor antagonist comprises sulpiride.

According to some embodiments of the invention, the method further comprises labeling the dopaminergic neurons with the fluorescent dopamine analog following step (c) and prior to step (d).

According to some embodiments of the invention, the agent does not bind to a dopamine receptor.

According to some embodiments of the invention, the agent is not transported through a dopamine transporter.

According to some embodiments of the invention, the inducing cells to differentiate is effected by contacting the pluripotent stem cells in a medium comprising FGF8, Purmorphamine and CHIR99021.

According to an aspect of some embodiments of the present invention there is provided a method of determining whether an agent effects dopaminergic differentiation, the agent not being capable of binding to a dopamine receptor or transported through a dopamine transporter, the method comprising:
(a) inducing cells to differentiate into dopaminergic neurons in the presence of the agent;
(b) labeling the dopaminergic neurons with a fluorescent dopamine analog;
(c) measuring a level of fluorescence in the dopaminergic neurons, wherein when the level is above a predetermined amount the agent is indicative as having a positive effect on dopaminergic differentiation.

According to some embodiments of the invention, the cells comprise pluripotent stem cells.

According to some embodiments of the invention, the pluripotent stem cells comprise embryonic stem (ES) cells.

According to some embodiments of the invention, the pluripotent stem cells comprise induced pluripotent stem (iPS) cells.

According to some embodiments of the invention, the labeling is effected in the presence of a dopamine receptor antagonist.

According to some embodiments of the invention, the fluorescent dopamine analog comprises Dansyl D1.

According to some embodiments of the invention, the dopamine receptor antagonist comprises sulpiride.

According to some embodiments of the invention, the inducing cells to differentiate is effected by contacting the pluripotent stem cells in a medium comprising FGF8, Purmorphamine and CHIR99021.

According to an aspect of some embodiments of the present invention there is provided a method of determining whether an agent is a neuroeffector, the agent not being capable of binding to a dopamine receptor or transported through a dopamine transporter, the method comprising:
(a) labeling dopaminergic neurons with a fluorescent dopamine analog to obtain fluorescing dopaminergic neurons;
(b) measuring a level of fluorescence in fluorescing dopaminergic neurons;
(c) exposing the fluorescing dopaminergic neurons to the agent;
(d) remeasuring a level of fluorescence in the fluorescing dopaminergic neurons, wherein a change in the level of fluorescence is indicative of the substance being a neuroeffector.

According to some embodiments of the invention, the dopaminergic neurons are generated by ex vivo differentiating pluripotent stem cells.

According to some embodiments of the invention, the pluripotent stem cells comprise embryonic stem (ES) cells.

According to some embodiments of the invention, the pluripotent stem cells comprise induced pluripotent stem (iPS) cells.

According to some embodiments of the invention, when the agent is a neurotoxin, the change in the level of fluorescence is a decrease.

According to some embodiments of the invention, when the agent is neurotrophic, the change in the level of fluorescence is an increase.

According to some embodiments of the invention, the labeling is effected in the presence of a dopamine receptor antagonist.

According to some embodiments of the invention, the fluorescent dopamine analog comprises Dansyl D1.

According to some embodiments of the invention, the dopamine receptor antagonist comprises sulpiride.

According to some embodiments of the invention, the method further comprises labeling the mixed population of cells with the fluorescent dopamine analog following step (c) and prior to step (d).

According to some embodiments of the invention, the ex vivo differentiating is effected by contacting the pluripotent stem cells in a medium comprising FGF8, Purmorphamine and CHIR99021.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a schematic representation of a protocol for differentiation of embryonic stem cells into dopaminergic neurons.
FIGs. 2A-C are photomicrographs illustrating that PC12 cells express PC-12 TH and dopamine transporter DAT.
FIGs. 3A-D are photomicrographs illustrating the effect of dopamine and GBR (a dopamine transporter antagonist) on fluorescence by Dansyl D1 in PC12 cells.
FIG. 4 is a bar graph illustrating the effect of different combinations of differentiation factors on the generation of dopaminergic neurons from ES cells.
FIGs. 5A-B are photomicrographs illustrating the labeling of dopaminergic neurons with Dansyl D1.
FIGs. 6A-D are photomicrographs illustrating the labeling of dopaminergic neurons with Dansyl D1 in the presence of dopamine (FIG. 6B) and GBR (FIG. 6C).
FIGs. 7A-C are photomicrographs illustrating that Dansyl D1 labels tyrosine hydroxylase (TH) expressing cells. FIG.7A illustrates labeling of dopaminergic neurons with Dansyl D1. FIG. 7B illustrates labeling of dopaminergic neurons with TH. FIG. 7C illustrates labeling of dopaminergic neurons with TH and Dansyl D1.
FIGs. 8A-B are photomicrographs illustrating the labeling of dopaminergic neurons with Dansyl D1 in the presence and absence of sulpiride.
FIGs. 9A-C are graphs illustrating the optimal concentration of Dansyl D1 to be used in an uptake assay.
FIGs. 10A-C illustrate that the amount of Dansyl D1 fluorescence in dopaminergic cells is reduced in the presence of the toxin - 6-OH dopamine.
FIG. 10D is a graph illustrating the amount of TH in dopaminergic cells in the presence of the toxin - 6-OH dopamine.
FIG. 11 is a graph illustrating that amount of Dansyl D1fluorescence in dopaminergic cells is reduced in correlation with the amount of toxin - 6-OH dopamine added to the cells.
FIG. 12 is a graph illustrating that cells expressing GDNF were protected from the neurotoxic agent 6-OH dopamine.

### DESCRIPTION OF SPECIFIC EMBODIMENTS OF THE INVENTION

The present invention, in some embodiments thereof, relates to a method of screening for dopaminergic neuroeffectors using fluorescent dopamine analogs.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Screening of large libraries for compounds with potential therapeutic effects for the treatment of Parkinson's disease and other dopaminergic related disease has not been performed in mammalian animal models due to high costs and time limitations. Hence, the development of a cell-based high-throughput screening (HTS) system to identify novel therapeutic compounds would be highly valuable.

The present inventors developed a novel system for the detection of functional live dopaminergic (DA) neurons *in vitro.* This system is based on the specific binding and uptake of the fluorescent ligand Dansyl D1 (dopamine labeled with dansyl molecule, Five-Photon Biochemical™) by dopamine transporter (DAT), expressed on the surface of DA neurons.

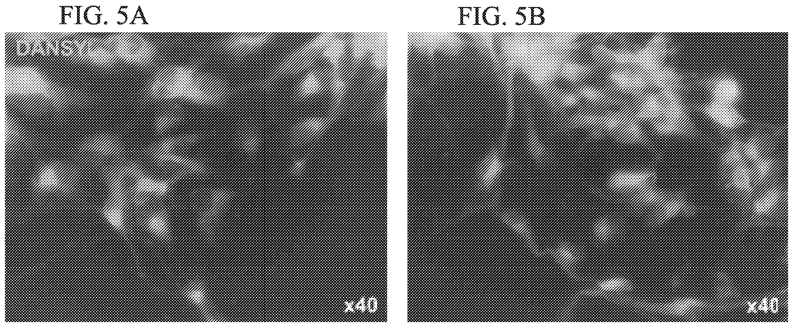

Whilst reducing the present invention to practice, the present inventors showed that they could label DA neurons which had been ex vivo differentiated from embryonic stem cells (Figures 5A-B).

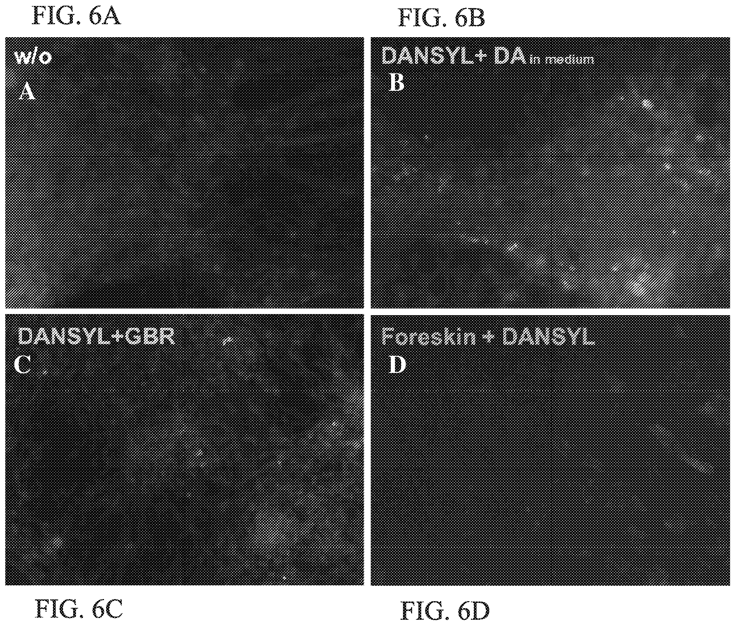

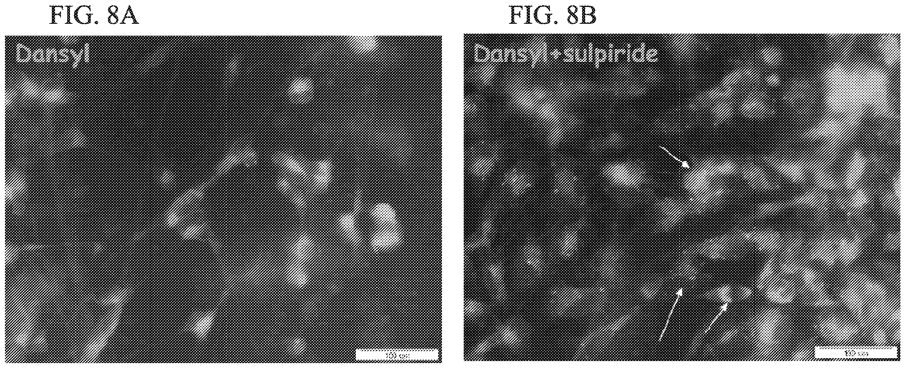

The present inventors showed that it is possible to selectively label dopaminergic cells using the fluorescent dopamine analog. The degree of labeling can be reduced by adding non-fluorescent dopamine and/or dopamine transporter blockers (as illustrated in Figures 6A-D) and/or dopamine receptor antagonists (Figures 8A-B).

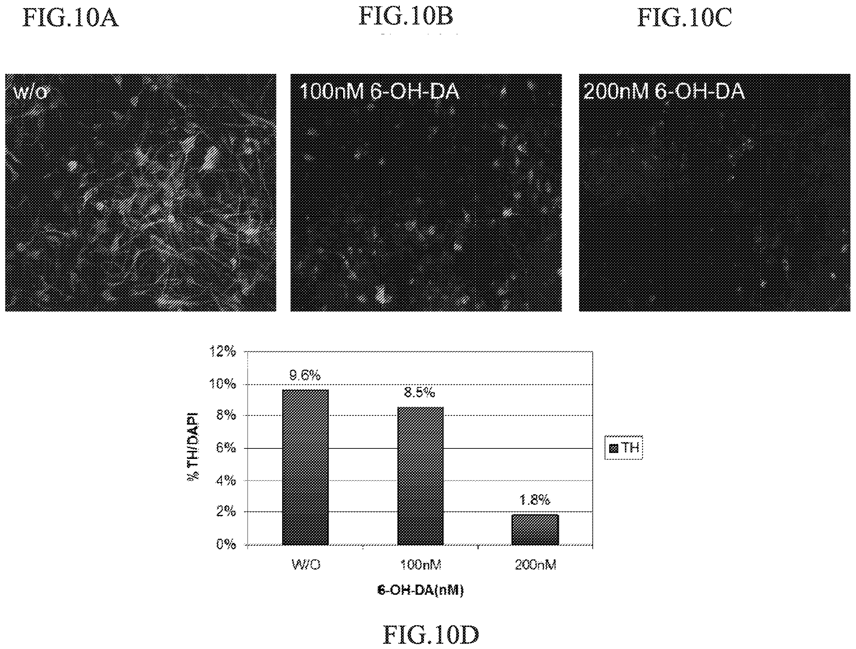

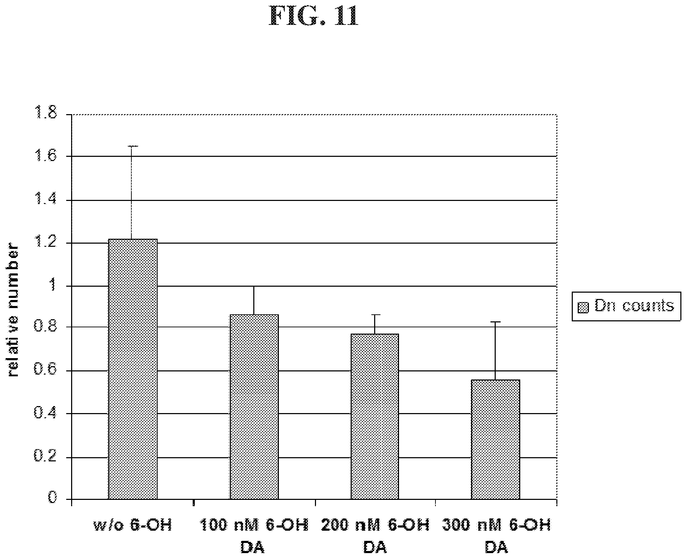

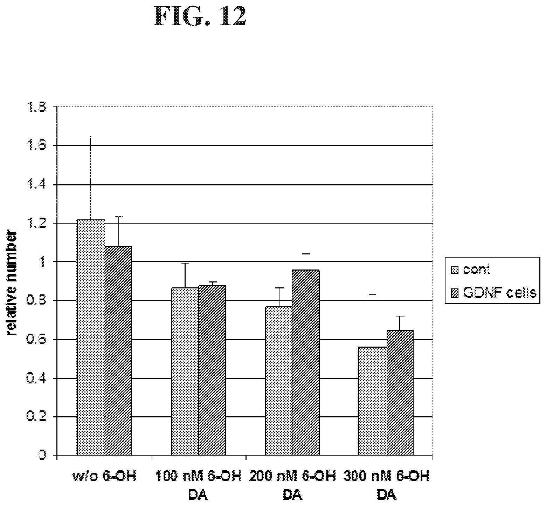

The degree of labeling was also reduced in a correlative manner in the presence of increasing amounts of dopaminergic toxin (Figures 10A-C and Figure 11). Further, the degree of labeling was increased in the presence of neurotrophic agents (Figure 12). These results indicate that fluorescent dopamine analogs can be used to screen for neuroeffectors in *in vitro* cell systems.

Thus, according to one aspect of the present invention there is provided a method of determining whether an agent is a neuroeffector, the method comprising:
(a) labeling dopaminergic neurons with a fluorescent dopamine analog to obtain fluorescing dopaminergic neurons;
(b) measuring a level of fluorescence in fluorescing dopaminergic neurons;
(c) exposing the fluorescing dopaminergic neurons to the agent;
(d) remeasuring a level of fluorescence in the fluorescing dopaminergic neurons, wherein a change in the level of fluorescence is indicative of the substance being a neuroeffector.

Below is a brief summary of dopaminergic transmission and involvement of the dopamine transporter (DAT).

***Dopamine transmission:*** Dopamine (DA), is a biogenic amine synthesized in the hypothalamus, in the arcuate nucleus, the caudate, and in other areas of the central and peripheral nervous system. Dopamine is also a precursor of other neurotransmitters, specifically, norepinephrine (NE) and epinephrine (E), in addition to being a neurotransmitter on its own. Dopamine and its agonists play important roles in cardiovascular, renal, hormonal, and central nervous system regulation through stimulation of alpha and beta adrenergic and dopaminergic receptors.

DA being a catechol and easily oxidized to a quinone, is often implicated as a generator of reactive oxygen species (ROS) like peroxide (H.sub.2O.sub.2), superoxide (O.sup.2-) and hydroxyl radical (OH).sup.- the latter being the most reactive and detrimental ROS. The dopamine transporter protein (DAT) is responsible for the uptake of excess dopamine that is released into the synaptic space back into neurons. Uptake of dopamine by DAT is important for regulating neuronal signaling as well as reducing the potential for DA being oxidized to form ROS.

***Dopamine Transporter Protein (DAT):*** DAT is a plasma membrane transport protein that controls extracellular DA concentrations, by recapture of DA that has been released during the process of neurotransmission, into nerve terminal. More recently, DAT has been recognized as a major target for various pharmacologically active drugs and environmental toxins (Miller, et al., 1999b).

As used herein, the term "neuroeffector" refers to an agent that has an effect on neuronal cells. The effect may be a toxic effect (e.g. decrease survival of neurons, or decrease the functioning of neurons) a protective effect, or a trophic effect (e.g. increase the survival of neurons, or increase the functioning of neurons). The effect may be an increase or decrease in neurotransmitter release (e.g. dopamine release), or neurotransmitter uptake (e.g. dopamine uptake). Alternatively, or additionally, the effect may be to increase (or decrease) the amount and/or activity or dopamine receptors and/or dopamine transporters.

Exemplary agents that may be tested include biological agents and/or chemical agents.

Examples of biological agents that may be tested as neuroeffectors according to the method of the present invention include, but are not limited to, nucleic acids, e.g., polynucleotides, ribozymes, siRNA and antisense molecules (including without limitation RNA, DNA, RNA/DNA hybrids, peptide nucleic acids, and polynucleotide analogs having altered backbone and/or bass structures or other chemical modifications); proteins, polypeptides (e.g. peptides), carbohydrates, lipids and "small molecule" drug candidates. "Small molecules" can be, for example, naturally occurring compounds (e.g., compounds derived from plant extracts, microbial broths, and the like) or synthetic organic or organometallic compounds having molecular weights of less than about 10,000 daltons, preferably less than about 5,000 daltons, and most preferably less than about 1,500 daltons.

The agent may be a potential therapeutic for the treatment of a particular disorder (a dopaminergic neuron related disorder of a dopaminergic neuron non-related disorder) and the agent may be tested according to the method described herein to ensure that it has no side effects on dopaminergic neurons.

According to a particular embodiment, the agent does not bind to the dopamine receptor and/or enter the cell through the dopamine transporter (DAT).

The term "dopamine receptor" as used herein refers to any of the five subtypes of dopamine receptors, D₁, D₂, D₃, D₄, and D₅. The D₁ and D₅ receptors are members of the D₁-like family of dopamine receptors, whereas the D₂, D₃ and D₄ receptors are members of the D₂-like family.

Examples of agents which bind to the dopamine receptor include dopamine receptor agonists and dopamine receptor antagonists.

As mentioned, the method is effected by labeling dopaminergic neurons with a fluorescent dopamine analog.

As used herein, the term "dopamine analog" refers to an agent that is capable of binding to the dopamine receptor and/or being transported through the dopamine transporter (DAT).

The dopamine analogs may be fluorescently labeled with a fluorescent moiety using standard labeling techniques known to and used by those of skill in the art.

The fluorescent moiety may be any red, green, near ir, blue or the like absorbing dyes or other class of dye. Suitably a fluorescent moiety is selected from dyes in particular including fluorescein, fluorescein derivatives including FITC, and fluorescein-like molecules such as Oregon Green.TM. and its derivatives, Texas red.TM., 7-nitrobenz-2-oxa-1,3-diazole (NBD) and derivatives thereof, coumarin and derivatives, naphthalene including derivatives of dansyl chloride or its analogues or derivatives, Cascade Blue.TM., EvoBlue and fluorescent derivatives thereof, pyrenes and pyridyloxazole derivatives, the cyanine dyes, the dyomics (DY dyes and ATTO dyes) and fluorescent derivatives thereof, the Alexafluor dyes and derivatives, BDI dyes including the commercially available Bodipy.TM. dyes, erythosin, eosin, pyrenes, anthracenes, acridines, fluorescent phycobiliproteins and their conjugates and fluoresceinated microbeads, Rhodamine and fluorescent derivatives thereof including Rhodamine Green.TM. including the tetramethylrhodamines, X-thodamines and Texas Red derivatives, and Rhodol Green.TM., coupled to amine groups using the isocyanate, succinimidyl ester or dichlorotriazinyl-reactive groups and other red, blue or green absorbing fluorescent dyes in particular red absorbing dyes as reviewed in Buschmann V et al, Bioconjugate Chemistry (2002), ASAP article.

The fluorescent moiety may be selected from fluorescein derivatives and fluorescein-like molecules such as Oregon Green.TM. and its derivatives, Texas red.TM., 7-nitrobenz-2-oxa-1,3-diazole (NBD) and derivatives thereof, coumarin and derivatives, naphthalene including derivatives of dansyl chloride or its analogues or derivatives, Cascade Blue.TM., EvoBlue and fluorescent derivatives thereof, pyrenes and pyridyloxazole derivatives, the cyanine dyes, the dionics (DY dyes and ATTO dyes) and fluorescent derivatives thereof, the Alexafluor dyes and derivatives, BDI dyes including the commercially available Bodipy.TM. dyes, erythosin, eosin, FITC, pyrenes, anthracenes, acridines, fluorescent phycobiliproteins and their conjugates and fluoresceinated microbeads, Rhodamine derivatives thereof including Rhodamine Green.TM. including the tetramethylrhodamines, X-rhodamines and Texas Red derivatives, and Rhodol Green.TM.

The fluorescent moiety may comprise fluorescein, Texas Red.TM., Cy5.5 or Cy5 or analogues thereof, BODIPY .TM. 630/650 and analogues thereof in particular BODIPY .TM. 630/650X, DY-630, DY-640, DY-650 or DY-655 or analogues thereof, ATTO 655 or ATTO 680 or analogues thereof, EvoBlue 30 or analogues thereof, Alexa 647 or analogues thereof.

Suitably a fluorescent moiety is derived from any of the above commercially available fluorophores, comprising or modified to comprise a reactive group facilitating linking to a ligand.

According to a particular embodiment the fluorescent dopamine is tailored by the site of linking of fluorescent and ligand moieties, the means of linking, i.e. nature and length of linker, and the stoichiometry thereof, i.e., 1:1, 2:1, 1:2 etc, whereby binding and function of the dopamine are retained in the fluorescent dopamine, and pharmacological properties are known whereby modulation of binding and function are known.

Commercially available dopamine analogs suitable for use in the compounds and methods of the present invention may include, but are not limited to SKF 38393 (DI-specific), Quinpirole (D2-specific), PD-168077 (D4-specific) (see: Research Biochemicals Incorporated, Nattick, Mass., USA) and 7-OH-DPAT (DPAT) (D3 specific).

In one preferred embodiment of the present invention, the Dopamine analog is a specific D2 receptor analog.

Examples of fluorescent dopamine analogs include for example DansylD1 (dopamine labeled with dansyl molecule, Five-Photon Biochemical™), JHC1-64, FFN511, 4-(4-diethylaminostyryl)-N-methylpyridinium iodide and those disclosed in U.S. Patent Nos. Nos. 6,420,183, 7,063,952, 7,138,280.

The dopaminergic neurons which are labeled may be a primary culture of dopaminergic neurons or may be generated using ex vivo differentiation techniques. According to one embodiment, the dopaminergic neurons are obtained by ex vivo differentiation of adult stem cells (e.g. neural stem cells or mesenchymal stem cells). According to another embodiment, the dopaminergic neurons are obtained by ex vivo differentiation of pluripotent stem cells, such as embryonic stem cells or induced pluripotent stem cells. According to still another embodiment, the dopaminergic neurons are obtained by ex vivo differentiation of mesenchymal stem cells.

According to a particular embodiment, the DA neurons are human neurons.

The phrase "dopaminergic cells" refers to neurons that release dopamine in response to electrical stimulation. Preferably, the dopaminergic cells express at least one dopaminergic marker such as a dopaminergic transcription factor such as Aldehyde dehydrogenase 1 (Aldh1), Engrailed 1(En-1), Nurr-1 or Paired-like homeodomain transcription factor 3 (PITX-3) or a dopaminergic protein such as Aromatic L-amino acid decarboxylase (AADC), Catechol-o-methyltransferase (COMT), Dopamine transporter (DAT), Dopamine receptor D2 (DRD2), GTP cyclohydrolase-1 (GCH), Monoamine oxidase B (MAO-B), Tryptophan hydroxylase (TPH), Vesicular monoamine transporter 2 (VMAT 2), Patched homolog (PTCH), Smoothened (SMO) or Tyrosine hydroxylase (TH).

The DA neurons may be comprised in a homogeneous population of cells (i.e. all the cells within the culture system are dopaminergic neurons) or may be comprised in a mixed population of cells (i.e. additional cells may be present in the culture system). The additional cells may be present because they have been retrieved in the brain biopsy together with the DA neurons. Such cells include serotonergic neurons, GABAergic neurons, astrocytes or oligodendrocytes. The additional cells may be present because the DA neurons have been generated through ex vivo differentiation of stem cells. It will be appreciated that not all the cells in the culture will have differentiated to the same extent and in the exact same way. Thus, the mixed cell population may comprise stem cells and partially differentiated stem cells towards the dopaminergic lineage.

According to one embodiment, the mixed population of cells comprises dopaminergic neurons and cells that express a dopamine receptor but do not express a dopamine transporter (e.g. GABAergic or adrenergic neurons).

As used herein, the phrase "stem cells" refers to cells which are capable of remaining in an undifferentiated state (e.g., pluripotent or multipotent stem cells) for extended periods of time in culture until induced to differentiate into other cell types having a particular, specialized function (e.g., fully differentiated cells). Preferably, the phrase "stem cells" encompasses embryonic stem cells (ESCs), induced pluripotent stem cells (iPS), adult stem cells and hematopoietic stem cells.

The phrase "embryonic stem cells" refers to embryonic cells which are capable of differentiating into cells of all three embryonic germ layers (*i.e.,* endoderm, ectoderm and mesoderm), or remaining in an undifferentiated state. The phrase "embryonic stem cells" may comprise cells which are obtained from the embryonic tissue formed after gestation (e.g., blastocyst) before implantation of the embryo (*i.e.,* a pre-implantation blastocyst), extended blastocyst cells (EBCs) which are obtained from a post-implantation/pre-gastrulation stage blastocyst (see WO2006/040763) and embryonic germ (EG) cells which are obtained from the genital tissue of a fetus any time during gestation, preferably before 10 weeks of gestation.

Induced pluripotent stem cells (iPS; embryonic-like stem cells), are cells obtained by de-differentiation of adult somatic cells which are endowed with pluripotency (*i.e.,* being capable of differentiating into the three embryonic germ cell layers, i.e., endoderm, ectoderm and mesoderm). According to some embodiments of the invention, such cells are obtained from a differentiated tissue (e.g., a somatic tissue such as skin) and undergo de-differentiation by genetic manipulation which re-program the cell to acquire embryonic stem cells characteristics. According to some embodiments of the invention, the induced pluripotent stem cells are formed by inducing the expression of Oct-4, Sox2, Kfl4 and c-Myc in a somatic stem cell.

The phrase "adult stem cells" (also called "tissue stem cells" or a stem cell from a somatic tissue) refers to any stem cell derived from a somatic tissue [of either a postnatal or a prenatal animal (especially the human)]. The adult stem cell is generally thought to be a multipotent stem cell, capable of differentiation into multiple cell types. Adult stem cells can be derived from any adult, neonatal or fetal tissue such as adipose tissue, skin, kidney, liver, prostate, pancreas, intestine, bone marrow and placenta.

Hematopoietic stem cells, which may also referred to as adult tissue stem cells, include stem cells obtained from blood or bone marrow tissue of an individual at any age or from cord blood of a newborn individual. Preferred stem cells according to this aspect of some embodiments of the invention are embryonic stem cells, preferably of a human or primate (e.g., monkey) origin.

Placental and cord blood stem cells may also be referred to as "young stem cells".

The embryonic stem cells of some embodiments of the invention can be obtained using well-known cell-culture methods. For example, human embryonic stem cells can be isolated from human blastocysts. Human blastocysts are typically obtained from human *in vivo* preimplantation embryos or from *in vitro* fertilized (IVF) embryos. Alternatively, a single cell human embryo can be expanded to the blastocyst stage. For the isolation of human ES cells the zona pellucida is removed from the blastocyst and the inner cell mass (ICM) is isolated by immunosurgery, in which the trophectoderm cells are lysed and removed from the intact ICM by gentle pipetting. The ICM is then plated in a tissue culture flask containing the appropriate medium which enables its outgrowth. Following 9 to 15 days, the ICM derived outgrowth is dissociated into clumps either by a mechanical dissociation or by an enzymatic degradation and the cells are then re-plated on a fresh tissue culture medium. Colonies demonstrating undifferentiated morphology are individually selected by micropipette, mechanically dissociated into clumps, and re-plated. Resulting ES cells are then routinely split every 4-7 days. For further details on methods of preparation human ES cells see Thomson et al., [U.S. Pat. No. 5,843,780; Science 282: 1145, 1998; Curr. Top. Dev. Biol. 38: 133, 1998; Proc. Natl. Acad. Sci. USA 92: 7844, 1995]; Bongso et al., [Hum Reprod 4: 706, 1989]; and Gardner et al., [Fertil. Steril. 69: 84, 1998].

It will be appreciated that commercially available stem cells can also be used according to some embodiments of the invention. Human ES cells can be purchased from the NIH human embryonic stem cells registry [Hypertext Transfer Protocol://grants (dot) nih (dot) gov/stem_cells/registry/current (dot) htm]. Nonlimiting examples of commercially available embryonic stem cell lines are BG01, BG02, BG03, BG04, CY12, CY30, CY92, CY10, TE03, TE32, CHB-4, CHB-5, CHB-6, CHB-8, CHB-9, CHB-10, CHB-11, CHB-12, HUES 1, HUES 2, HUES 3, HUES 4, HUES 5, HUES 6, HUES 7, HUES 8, HUES 9, HUES 10, HUES 11, HUES 12, HUES 13, HUES 14, HUES 15, HUES 16, HUES 17, HUES 18, HUES 19, HUES 20, HUES 21, HUES 22, HUES 23, HUES 24, HUES 25, HUES 26, HUES 27, HUES 28, CyT49, RUES3, WA01, UCSF4, NYUES1, NYUES2, NYUES3, NYUES4, NYUES5, NYUES6, NYUES7, UCLA 1, UCLA 2, UCLA 3, WA077 (H7), WA09 (H9), WA13 (H13), WA14 (H14), HUES 62, HUES 63, HUES 64, CT1, CT2, CT3, CT4, MA135, Eneavour-2, WIBR1, WIBR2, WIBR3, WIBR4, WIBR5, WIBR6, HUES 45, Shef 3, Shef 6, BJNhem19, BJNhem20, SA001, SA001.

In addition, ES cells can be obtained from other species as well, including mouse (Mills and Bradley, 2001), golden hamster [Doetschman et al., 1988, Dev Biol. 127: 224-7], rat [Iannaccone et al., 1994, Dev Biol. 163: 288-92] rabbit [Giles et al. 1993, Mol Reprod Dev. 36: 130-8; Graves & Moreadith, 1993, Mol Reprod Dev. 1993, 36: 424-33], several domestic animal species [Notarianni et al., 1991, J Reprod Fertil Suppl. 43: 255-60; Wheeler 1994, Reprod Fertil Dev. 6: 563-8; Mitalipova et al., 2001, Cloning. 3: 59-67] and non-human primate species (Rhesus monkey and marmoset) [Thomson et al., 1995, Proc Natl Acad Sci U S A. 92: 7844-8; Thomson et al., 1996, Biol Reprod. 55: 254-9].

Extended blastocyst cells (EBCs) can be obtained from a blastocyst of at least nine days post fertilization at a stage prior to gastrulation. Prior to culturing the blastocyst, the zona pellucida is digested [for example by Tyrode's acidic solution (Sigma Aldrich, St Louis, MO, USA)] so as to expose the inner cell mass. The blastocysts are then cultured as whole embryos for at least nine and no more than fourteen days post fertilization (*i.e.,* prior to the gastrulation event) *in vitro* using standard embryonic stem cell culturing methods.

Another method for preparing ES cells is described in Chung et al., Cell Stem Cell, Volume 2, Issue 2, 113-117, 7 February 2008. This method comprises removing a single cell from an embryo during an in vitro fertilization process. The embryo is not destroyed in this process.

EG cells are prepared from the primordial germ cells obtained from fetuses of about 8-11 weeks of gestation (in the case of a human fetus) using laboratory techniques known to anyone skilled in the arts. The genital ridges are dissociated and cut into small chunks which are thereafter disaggregated into cells by mechanical dissociation. The EG cells are then grown in tissue culture flasks with the appropriate medium. The cells are cultured with daily replacement of medium until a cell morphology consistent with EG cells is observed, typically after 7-30 days or 1-4 passages. For additional details on methods of preparation human EG cells see Shamblott et al., [Proc. Natl. Acad. Sci. USA 95: 13726, 1998] and U.S. Pat. No. 6,090,622.

Induced pluripotent stem cells (iPS) (embryonic-like stem cells) can be generated from somatic cells by genetic manipulation of somatic cells, e.g., by retroviral transduction of somatic cells such as fibroblasts, hepatocytes, gastric epithelial cells with transcription factors such as Oct-3/4, Sox2, c-Myc, and KLF4 [Yamanaka S, Cell Stem Cell. 2007, 1(1):39-49; Aoi T, et al., Generation of Pluripotent Stem Cells from Adult Mouse Liver and Stomach Cells. Science. 2008 Feb 14. (Epub ahead of print); IH Park, Zhao R, West JA, et al. Reprogramming of human somatic cells to pluripotency with defined factors. Nature 2008;451:141-146; K Takahashi, Tanabe K, Ohnuki M, et al. Induction of pluripotent stem cells from adult human fibroblasts by defined factors. Cell 2007;131:861-872]. Other embryonic-like stem cells can be generated by nuclear transfer to oocytes, fusion with embryonic stem cells or nuclear transfer into zygotes if the recipient cells are arrested in mitosis.

Adult tissue stem cells can be isolated using various methods known in the art such as those disclosed by Alison, M.R. [J Pathol. 2003 200(5): 547-50], Cai, J. et al., [Blood Cells Mol Dis. 2003 31(1): 18-27], Collins, A.T. et al., [J Cell Sci. 2001; 114(Pt 21): 3865-72], Potten, C. S. and Morris, R. J. [Epithelial stem cells in vivo. 1988. J. Cell Sci. Suppl. 10, 45-62], Dominici, M et al., [J. Biol. Regul. Homeost. Agents. 2001, 15: 28-37], Caplan and Haynesworth [U.S. Pat. No. 5,486,359] Jones E.A. et al., [Arthritis Rheum. 2002, 46(12): 3349-60]. Fetal stem cells can be isolated using various methods known in the art such as those disclosed by Eventov-Friedman S, et al., PLoS Med. 2006, 3: e215; Eventov-Friedman S, et al., Proc Natl Acad Sci U S A. 2005, 102: 2928-33; Dekel B, et al., 2003, Nat Med. 9: 53-60; and Dekel B, et al., 2002, J. Am. Soc. Nephrol. 13: 977-90. Hematopoietic stem cells can be isolated using various methods known in the arts such as those disclosed by "Handbook of Stem Cells" edit by Robert Lanze, Elsevier Academic Press, 2004, Chapter 54, pp609-614, isolation and characterization of hematopoietic stem cells, by Gerald J Spangrude and William B Stayton.

Generally, isolation of adult tissue stem cells is based on the discrete location (or niche) of each cell type included in the adult tissue, *i.e.,* the stem cells, the transit amplifying cells and the terminally differentiated cells [Potten, C. S. and Morris, R. J. (1988). Epithelial stem cells in vivo. J. Cell Sci. Suppl. 10, 45-62]. Thus, an adult tissue such as, for example, prostate tissue is digested with Collagenase and subjected to repeated unit gravity centrifugation to separate the epithelial structures of the prostate (e.g., organoids, acini and ducts) from the stromal cells. Organoids are then disaggregated into single cell suspensions by incubation with Trypsin/EDTA (Life Technologies, Paisley, UK) and the basal, CD44-positive, stem cells are isolated from the luminal, CD57-positive, terminally differentiated secretory cells, using anti-human CD44 antibody (clone G44-26; Pharmingen, Becton Dickinson, Oxford, UK) labeling and incubation with MACS (Miltenyi Biotec Ltd, Surrey, UK) goat anti-mouse IgG microbeads. The cell suspension is then applied to a MACS column and the basal cells are eluted and re-suspended in WAJC 404 complete medium [Robinson, E.J. et al. (1998). Basal cells are progenitors of luminal cells in primary cultures of differentiating human prostatic epithelium Prostate 37, 149-160].

Since basal stem cells can adhere to basement membrane proteins more rapidly than other basal cells [Jones, P.H. et al. (1995). Stem cell patterning and fate in human epidermis. Cell 60, 83-93; Shinohara, T., et al. (1999). β1- and α6-integrin are surface markers on mouse spermatogonial stem cells. Proc. Natl. Acad. Sci. USA 96, 5504-5509] the CD44 positive basal cells are plated onto tissue culture dishes coated with either type I collagen (52 µg/ml), type IV collagen (88 µg/ml) or laminin 1 (100 µg/ml; Biocoat®, Becton Dickinson) previously blocked with 0.3 % bovine serum albumin (fraction V, Sigma-Aldrich, Poole, UK) in Dulbecco's phosphate buffered saline (PBS; Oxoid Ltd, Basingstoke, UK). Following 5 minutes, the tissue culture dishes are washed with PBS and adherent cells, containing the prostate tissue basal stem cells are harvested with trypsin-EDTA.

Methods of differentiating stem cells into dopaminergic neurons are known in the art and include culturing in a differentiating medium which comprises differentiating agents. Alternatively and/or additionally, the stem cells may be genetically modified to express a protein known to induce dopaminergic differentiation.

Differentiation to dopaminergic cells can be effected by incubating the cells in differentiating media such as those described in U.S. Pat. No. 6,528,245 and by Sanchez-Ramos *et al.* (2000); Woodburry *et al.* (2000); Woodburry et al. (J. Neurisci. Res. 96:908-917, 2001); Black and Woodbury (Blood Cells Mol. Dis. 27:632-635, 2001); Deng *et al.* (2001), Kohyama *et al.* (2001), Reyes and Verfatile (Ann. N. Y. Acad. Sci. 938:231-235, 2001) and Jiang et al. (Nature 418:47-49, 2002).

Exemplary agents that may be used for differentiating stem cells include for example BHA, ascorbic acid, BDNF, GDNF, NT-3, IL-1β, NTN, TGFβ3 and dbcAMP, PUFA, FGF-1, FGF-17, SHH, IBMX, forskolin, noggin, PMA/TPA, dopamine, SMAD inhibitors (LDN193189+SB431542), FGF8, Purmorphamine (SHH agonists or modified SHH molecules), BIO, Wnt1 and CHIR99021.

According to one embodiment the agents include FGF8, Purmorphamine, SMAD inhibitors (LDN193189 +SB431542) and CHIR99021.

According to a particular embodiment the agents include FGF8 (e.g. 25-200 ng/ml, 25-100 ng/ml, 50-100 ng/ml)) Purmorphamine (0.25-1 µM) and CHIR99021 (1-5 µM).

Methods of differentiating embryonic stem cells into dopaminergic neurons are disclosed in U.S. Patent No. 7,604,992. According to a particular embodiment, when pluripotent stem cells are used as the source of DA neurons, the agents FGF8, Purmorphamine and CHIR99021 are used.

According to one embodiment, the dopaminergic neurons are differentiated from embryonic stem cells via the generation of embryoid bodies. As used herein the phrase "embryoid bodies" (EBs) refers to three dimensional multicellular aggregates of differentiated and undifferentiated cells derivatives of three embryonic germ layers.

Embryoid bodies are formed upon the removal of ES cells from feeder layers or feeder cells-free culture systems. ES cells removal can be effected using type IV Collagenase treatment for a limited time. Following dissociation from the culturing surface, the cells are transferred to tissue culture plates containing a culture medium supplemented with serum and amino acids.

During the culturing period, EBs are further monitored for their differentiation state. Cell differentiation can be determined upon examination of cell or tissue-specific markers which are known to be indicative of differentiation. For example, EB-derived-differentiated cells may express the neurofilament 68 KD which is a characteristic marker of the ectoderm cell lineage.

The differentiation level of the EB cells can be monitored by following the loss of expression of Oct-4, and the increased expression level of other markers such as α-fetoprotein, NF-68 kDa, α-cardiac and albumin.

As mentioned, the stem cells may be genetically modified so as to induce them to release dopamine. Exemplary enzymes that may be expressed in the stem cells in order to induce DA differentiation include for example tyrosine hydroxylase, DOPA decarboxylase, GTP cyclohydrolase I, dopamine β-hydroxylase, glutamate decarboxylase, tryptophane-5 monooxygenase and choline acetyltransferase.

The present inventors further contemplate differentiation of cells towards a dopaminergic lineage by genetically modifying them to express a polynucleotide agent (e.g. siRNA or miRNA).

As mentioned, once DA neurons are obtained they are labeled with the fluorescent dopamine analog. The DA neurons are contacted with the analog under conditions (e.g. for sufficient time and at the appropriate temperature) that allows the analog to bind to and/or enter the neurons.

According to one embodiment, the labeling is effected in the presence of a dopamine receptor antagonist. Preferably, the dopamine receptor antagonist is a D2 receptor antagonist.

Examples of dopamine receptor antagonists include, but are not limited to acepromazine, amisulpride, amoxapine, azaperone, benperidol, bromopride, butaclamol, clomipramine, chlorpromazine, chlorprothixene, clopenthixol, domperidone, droperidol, eticlopride, flupenthixol, fluphenazine, fluspirilene, haloperidol, hydroxyzine, iodobenzamide, loxapine, mesoridazine, levomepromazine, metoclopramide, nafadotride, nemonapride, olanzapine, penfluridol, perazine, perphenazine, pimozide, prochlorperazine, promazine, raclopride, remoxipride, risperidone, spiperone, spiroxatrine, stepholidine, sulpiride, sultopride, tetrahydropalmatine, thiethylperazine, thioridazine, thiothixene, tiapride, trifluoperazine, trifluperidol, triflupromazine, ziprasidone.

According to a particular embodiment, the antagonist is sulpiride.

Following the labeling procedure, the DA are typically treated to remove any unbound fluorescent dopamine analog.

Fluorescence may be quantitated with any of the many devices known to those of ordinary skill in the art, including, but not limited to photomultipliers, photometers, fluorimeters, CCD-based cameras or optic fiber systems and using fluorescent microscopy. Alternatively, fluorescence may be quantitated by the naked eye with or without the use of a microscope system. Fluorescence may be quantitated in arbitrary units.

Following quantization of the fluorescent label, the dopaminergic neurons are contacted with the test agent. The test agent should be contacted with the cells for sufficient time (e.g. 1 hour, 2 hours, 3 hours, 4 hours, 5 hours 6 hours, 12 hours, 14 hours or 48 hours) and under conditions such that the agent can have induce an effect in the cells.

If the cells are contacted with the test agent for an amount of time which affects the strength of the fluorescent label, the cells are typically exposed again to the fluorescent dopamine analog. Preferably, the identical amount of fluorescent dopamine analog is added to the culture system before and after contacting with the agent.

The amount of fluorescence is measured again and the change in fluorescence before and after exposure to the agent is calculated. An increase in fluorescence indicates a trophic effect on the DA cells, whereas a decrease in fluorescence indicates a toxic effect on the DA cells. Preferably the change is fluorescence is at least 50 %, at least 40 %, at least 30 %, at least 20 % or even at least 10 %.

A control experiment may be performed concurrently wherein no test agent is added to the dopaminergic cells to ensure that the test conditions themselves do not have any adverse or positive effect on the cells.

The method described herein above may be used as an initial screen to identify dopaminergic neurotrophic agents for the treatment of dopaminergic diseases such as Parkinson's disease. Alternatively, the method described herein above may be used as a pharmatoxicology screen to identify whether therapeutic agents (or potential therapeutic agents being developed) have an adverse effect on dopaminergic neurons. It will be appreciated that as well as pharmacological agents, environmental agents and/or conditions may also be tested using the methods described herein.

Additional diseases that are connected to dopaminergic transmission for which the agents may be useful include Alzheimers Disease, Wilson's Disease, Lesch-Nylan Disease, Tourette's Syndrome, schizophrenia and chronic substance abusers.

The present inventors propose that the assay described herein above may be adapted so as to screen for agents which affect differentiation of cells (e.g. stem cells) towards the dopaminergic lineage.

Thus, according to another aspect of the present invention there is provided a method of determining whether an agent effects dopaminergic differentiation comprising:
(a) inducing cells to differentiate into dopaminergic neurons in the presence of the agent;
(b) labeling the dopaminergic neurons with a fluorescent dopamine analog;
(c) measuring a level of fluorescence in the dopaminergic neurons, wherein when the level is above a predetermined amount the agent is indicative as having a positive effect on dopaminergic differentiation.

The induction of differentiation may be effected by culturing cells in a differentiation medium and/or by genetically modifying them to express a protein as further described herein above.

According to this aspect of the present invention, the amount of fluorescence is measured following the differentiation protocol. If the amount of fluorescence is at least 5 % higher, at least 10 % higher, at least 20 % higher, at least 30 % higher than the amount of fluorescence in the dopaminergic neurons which have been differentiated using the same protocol but in the absence of the test agent, then the agent may be considered to have a positive effect on dopaminergic differentiation. If the amount of fluorescence is at least 5 % lower, at least 10 % lower, at least 20 % lower, at least 30 % lower than the amount of fluorescence in the dopaminergic neurons which have been differentiated using the same protocol but in the absence of the test agent, then the agent may be considered to have a negative effect on dopaminergic differentiation.

It is expected that during the life of a patent maturing from this application many relevant dopamine analogs and fluorescent moieties will be developed and the scope of the term fluorescent dopamine analog is intended to include all such new technologies *a priori.*

As used herein the term "about" refers to ± 10 %.

The terms "comprises", "comprising", "includes", "including", "having" and their conjugates mean "including but not limited to".

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### MATERIALS AND METHODS

### hESC maintenance

HES-1 cells were maintained on human foreskin fibroblasts treated for 2.5 hours with 10µg/ml mitomycin-C (Sigma, St. Louis, MO), and plated in gelatin-coated 9.5 cm² well plates (Nunc, Glostrup, Denmark; 3x10⁵ feeders/well). HES-1 cells were routinely cultured in 85 % knockout DMEM medium supplemented with 14 % knockout serum replacement, 1mM L-glutamine, 1% nonessential amino acids (10mM of each amino acid), 50U/ml penicillin, 50 µg/ml streptomycin, (all from Gibco, Carlsbad, CA) and 4 ng/ml basic fibroblast growth factor (bFGF, Cytolab, Rehovot, Israel). The medium was changed every day. The cells were passaged weekly as small clusters following digestion with Collagenase type IV (1mg/ml, Gibco) for 1 hour.

### Derivation of neural progenitors and controlled conversion of the neurospheres into dopaminergic neurons

In order to direct the differentiation of hESCs into DA neurons, the cells were first directed to become neural progenitors within free-floating spheres (2 weeks) in the presence of the dual SMAD inhibitors (LDN193189+SB431542), FGF8, Purmorphamine and CHIR. They were further directed to differentiate into DA progenitors (7 days) as adherent cultures on laminin/fibronectin in the presence of FGF8, Purmorphamine and CHIR. Finally, they were differentiated into mature DA neurons (7 days) in the presence of the following factors (BDNF, NT4, AA, db-cAMP, TGFbeta3, GDNF and DAPT). The relevant factors/mitogens were replaced every 2-3 days.

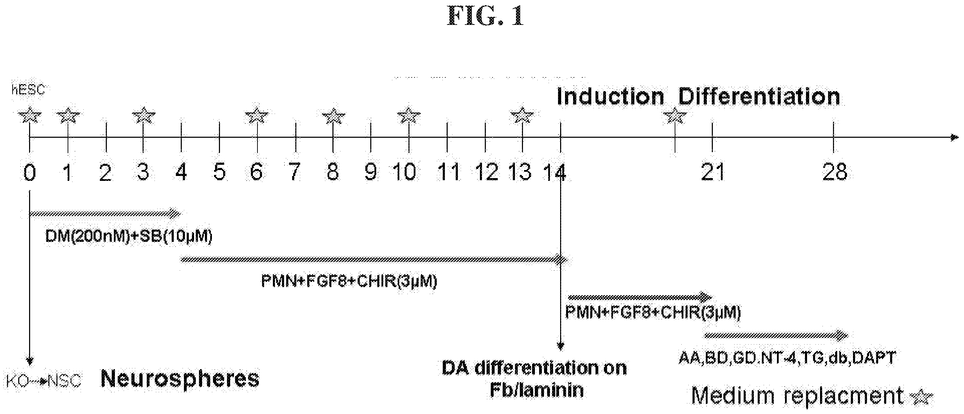

Schematic presentation of the DA neuron differentiation protocol is presented in Figure 1.

### Immunostaining of cells on coverslips

Neural progenitors from 2 week neurospheres were seeded onto cover slips (150,000-200,000 cells per 13 mm coverslip) pre-coated with poly-D-lysine (10 ug/ml), laminin (4 ug/ml) and fibronectin (2 µg/ml) and cultured in NB medium containing Purmorphamine FGF8 and CHIR as above. Seeded cells were fixed with 4% paraformaldehyde (PFA) after 7 days of induction and differentiation for an additional week in the presence of survival factors.

For staining, cells on the coverslips were blocked in 5% normal goat/donkey serum (NGS) for 1 hour at room temperature. The anti TH antibody was diluted 1:100 or 1:1000 in 1% normal donkey/goat serum (NGS) and applied for 1 hour at room temperature. The antibodies that was employed were rabbit anti-human TH polyclonal antibody or mouse anti-human TH monoclonal antibody, Cat No. p40101, purchased from Pel-freez, or Cat No. T1299 purchased from Sigma and rabbit anti hu Nurr1 ab AB5778 (Millipore 10ug/ml) or mouse igG anti NurrI (millipore, 1:200 or 1:1000), Rabbit Anti DAT AB5802 (Millipore, 1:500-1:1000). Following three washes in PBS, swine-anti rabbit FITC or goat anti mouse Cy-3- antibody diluted 1:50 or 1:500 in 1% normal goat serum (NGS) was applied for 1 hour at room temperature. Cells were washed in PBS, fixed with 4% PFA and mounted in the presence of the nuclear counterstain DAPI for immunofluoresecent microscopic examination.

### Quantification of the binding and uptake of the fluorescent ligand DansylD1

For Binding and uptake of the fluorescent ligand, 500-1000nM of DansylD1™ (Five Photon Biochemicals™, Lot#81672) was administrated to the seeded cells after 2 weeks of differentiation on the coverslips or on 12/24 wells plastic plates for 10-15 minutes in 37 °C. DansylD1 functions as the dopamine neurotransmitter in binding to the D2 dopamine receptor on Dopaminergic and GABAergic neurons and uptake through the dopamine transporter (DAT) by dopaminergic neurons. 50 nM of the D2 dopamine receptor antagonist - Sulpiride (sigma S112, Lot#108H4745) or 5 nM of the dopamine transporter (DAT) blocker - GBR (Sigma G9659, Lot#086K4104), were added for 10 minutes to the seeded cells in order to reduce the binding to non-dopaminergic neurons or to block the specific DansylD1 uptake respectively. Uptake levels of the dansylD1 fluorescent ligand molecule were detected and analyzed by fluorescence microscope and a fluorescence micro-plate reader (Bioteck, 333/515 nm). Acquisition and analysis was performed using Magelan software and Microsoft Excel.

### RESULTS

### Calibration and qualification of the DansylD1

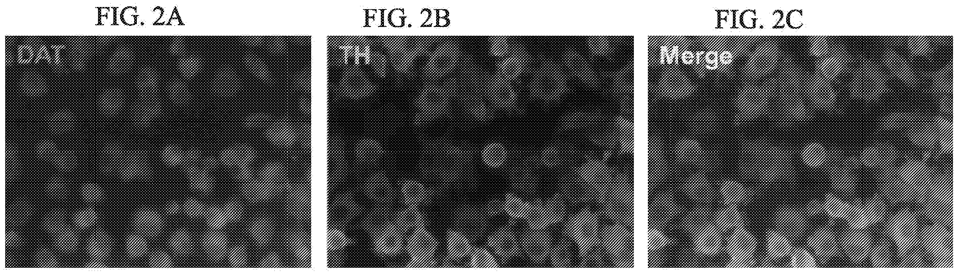

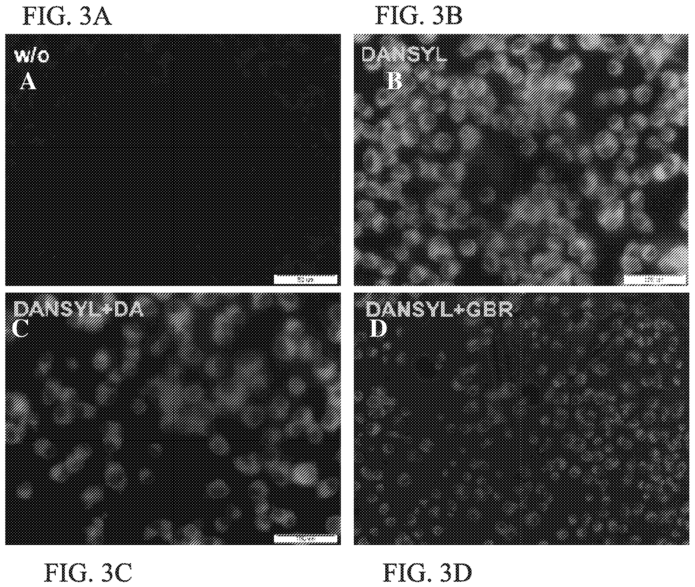

In order to calibrate the Dansyl D1 the PC-12 cell line was used as a model for dopaminergic neurons that express TH and dopamine transporter (Figures 2A-C). Following addition of 500 nM of DansylD1, the PC12 cells were detected in the flouorescence microscope. Dopamine was used as a competitive inhibitor of DansylD 1 uptake, whilst GBR was used as a dopamine transporter blocker. As illustrated in Figures 3A-D, both dopamine and GBR decreased the amount of fluorescence in the cells.

### Differentiation of hESCs to dopaminergic neurons

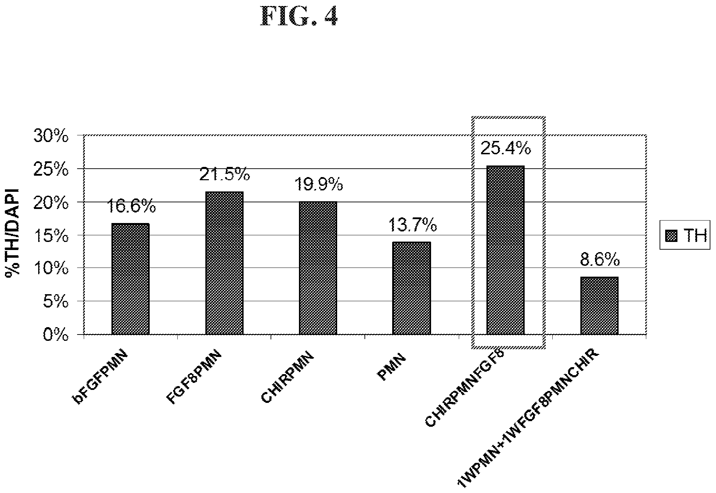

To examine the most efficient differentiation protocol to differentiate ES cells towards dopaminergic neurons, a number of possible combinations of differentiation were examined and tyrosine hydroxylase (TH), the key enzyme in dopamine synthesis was chosen as a marker for dopaminergic neurons. As illustrated in Figure 4, 25% percent of cells expressed TH treated following treatment with FGF8, Purmorphanime and CHIR.

### DansylD1 specifically labeled hESC-derived dopaminergic neurons

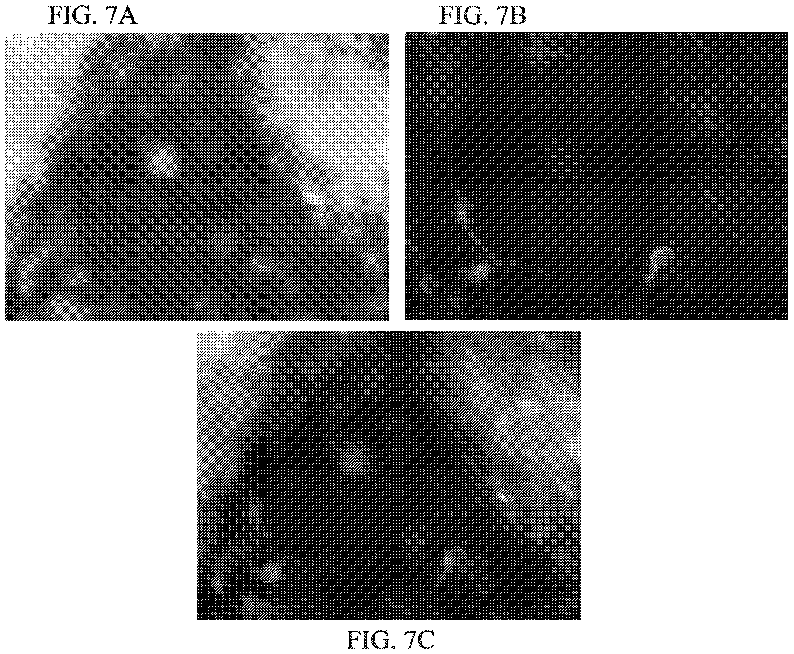

After 26 days of differentiation on fb/laminin coverslips, DansylD1 was added to the differentiated cells and the dopaminergic neurons were labeled with the fluorescent green stain (Figures 5A-B). As illustrated in Figures 6A-D, the presence of dopamine in the medium or GBR, a dopamine transporter blocker caused the fluorescence level to decline. Foreskin cells were used as a negative control (Figure 6D). The DansylD1 specifically labeled hESC-derived differentiated cells which were immunoreactive with anti-tyrosine hydroxylase (TH, red stain) as illustrated in Figures 7A-C. Specific blockers were added to reduce nonspecific background fluorescence related to binding of DansylD1 to dopamine receptor D2, which is not exclusively expressed on DA neurons.

The neuronal culture was incubated in the presence of the D2 antagonist Sulpiride. As illustrated in Figures 8A-B in the presence of sulpiride (8B) there is less signal from the cells surface, where the D2 receptor resides, and there is an appearance of fluorescent vesicles which indicate the specific uptake of Dansyl D1 through the dopamine transporter into the DA neuron cytosol and then into the dopaminergic vesicles via the vesicle monoamine transporter (VMAT).

### Calibration of the uptake levels of the DansylD1 fluorescent ligand molecule by DA mature neurons.

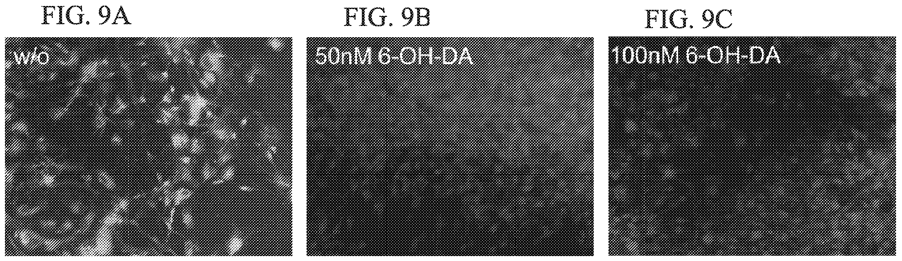

DansylD1 ligand uptake assay was calibrated on a microplate fluorescent reader either by adding DansylD1 in different concentrations to a single concentration of cells or by adding a known concentration of dansylD1 to different concentrations of cells. Figures 9A-B illustrate that 400,000 cells should be seeded on the cover slips and 500-1000 nM of dansyl should be added to the cells in order to receive appropriate signal.

### DansylD1fluorescence is reduced in the presence of the toxin 6-OH dopamine.

Dopaminergic neurons were fluorescently labeled with Dansyl D1. Increasing levels of 6-OH dopamine (50 nM and 100 nM) were added to the dopaminergic neurons and following 1 day of culture, the neurons were contacted again with Dansyl D1. The cells were rinsed and then the amount of fluorescence was analyzed.

Figures 10A-C illustrate that the amount of Dansyl D1 fluorescence in dopaminergic cells is reduced in the presence of the toxin - 6-OH dopamine.

An additional experiment compared the effect of 100 nM 6-OH dopamine with 200 nM 6-OH dopamine. As illustrated in Figures 11A-B, after addition of 200nM 6-OH-dopamine to mature DA neurons the fluorescence levels of dansylD1 was decreased (green) and also the percentage of cells expressing TH (DA neuron marker) declined to 1-2% (Figures 11C and D). Interestingly, a more moderate level of 100nM 6-OH-dopamine (Figure 11B and D) caused a significant decrease in Dansyl-Dl uptake but only minor reduction in TH positive cells, indicating Dansyl-D1 can detect neurotoxicity when cytotoxicity is not apparent.

### Quantification of the uptake levels of the DansylD1 fluorescent ligand molecule by DA mature neurons.

Dansyl D1 was added to 2 week differentiated live mature DA neurons and the fluorescence levels quantified by fluorescence micro-plate reader. Later the cells were washed and different 6-OH-dopamine concentrations were added. After a day, dansylD1 was added again and the fluorescent levels measured once again. After addition of the toxin 6-OH-dopamine the fluorescence levels were decreased by half (Figure 11).

As illustrated in Figure 12, cells over-expressing GDNF protected DNA neurons from the toxin.

Although the invention has been described in conjunction with specific embodiments thereof, it is evident that many alternatives, modifications and variations will be apparent to those skilled in the art. Accordingly, it is intended to embrace all such alternatives, modifications and variations that fall within scope of the appended claims.

### References

1. Nass R, Hall DH, Miller DM 3rd, Blakely RD. 2002. Neurotoxin-induced degeneration of dopamine neurons in Caenorhabditis elegans. Proc Natl Acad Sci USA 99:3264-3269.
2. Kovtun O, Ross EJ, Tomlinson ID, Rosenthal SJ. 2012. A flow cytometry-based dopamine transporter binding assay using antagonist-conjugated quantum dots. Chem Commun (Camb). 2012 Jun 4;48(44):5428-30. doi: 10.1039/c2cc31951a. Epub 2012 Apr 27.
3. Cha JH, Zou MF, Adkins EM, Rasmussen SG, Loland CJ, Schoenenberger B, Gether U, Newman AH. 2009. Rhodamine-labeled 2beta-carbomethoxy-3beta-(3,4-dichlorophenyl) tropane analogues as high-affinity fluorescent probes for the dopamine transporter. J Med Chem. 2005 48(24):7513-6.
4. Eriksen J, Rasmussen SG, Rasmussen TN, Vaegter CB, Cha JH, Zou MF, Newman AH, Gether U. 2009. Visualization of dopamine transporter trafficking in live neurons by use of fluorescent cocaine analogs. J. Neurosci. 2009 May 27;29(21):6794-808.
5. Gubernator NG, Zhang H, Staal RG, Mosharov EV, Pereira DB, Yue M, Balsanek V, Vadola PA, Mukherjee B, Edwards RH, Sulzer D, Sames D. 2009. Fluorescent false neurotransmitters visualize dopamine release from individual presynaptic terminals. Science. 324:1441-4.
6. Mason JN, Farmera H, Tomlinsonb ID, Schwartza JW, Savchenkoa V, DeFelicea LJ, Rosenthal SJ, Blakelya RD. 2005. Novel fluorescence-based approaches for the study of biogenic amine transporter localization, activity, and regulation. J. Neuroscience Methods 143:3-25.
7. Jørgensen S, Nielsen EØ, Peters D, Dyhring T. 2008. Validation of a fluorescence-based high-throughput assay for the measurement of neurotransmitter transporter uptake activity. J Neurosci Methods. 2008 169:168-76.
8. Reubinoff BE, Pera MF, Fong CY, Trounson A, and Bongso A. Embryonic stem cell lines from human blastocysts: somatic differentiation in vitro. 2000. Nature Biotechnology 18:399-404.
9. Reubinoff BE, Itsykson P, Turetsky T, Pera MF, Reinhartz E, Itzik A, Ben-Hur T. 2001. Neural progenitors from human embryonic stem cells. Nat Biotechnol. 19(12):1134-40.
10. Redman PT, Jefferson BS, Ziegler CB, Mortensen OV, Torres GE, Levitan ES, Aizenman E. 2006. A vital role for voltage-dependent potassium channels in dopamine transporter-mediated 6-hydroxydopamine neurotoxicity. Neuroscience. 143(1):1-6.
11. May S, Andreasson-Ochsner M, Fu Z, Low YX, Tan D, de Hoog HP, Ritz S, Nallani M, Sinner EK. 2013. In Vitro Expressed GPCR Inserted in Polymersome Membranes for Ligand-Binding Studies. Angew. Chem. Int. Ed. 2013, 52, 749-753

## Claims

1. An in-vitro method of determining whether an agent is a neuroeffector, the method comprising:
(a) labeling dopaminergic neurons which are comprised in a mixed population of cells with a fluorescent dopamine analog in the presence of a dopamine receptor antagonist, wherein said dopamine receptor antagonist comprises Sulpiride or an addition of the dopamine transporter blocker GBR;
(b) measuring a level of fluorescence in said mixed population of cells;
(c) exposing said mixed population of cells to said agent;
(d) remeasuring a level of fluorescence in said mixed population of cells, wherein a change in said level of fluorescence is indicative of the substance being a neuroeffector,
wherein said fluorescent dopamine analogue comprises a dopamine labelled with dansyl molecule.

2. The method of claim 1, wherein said mixed population of cells further comprises cells which express a dopamine receptor and do not express a dopamine transporter.

3. The method of claim 1, wherein said dopaminergic neurons are generated by ex vivo differentiating pluripotent stem cells.

4. The method of claim 1, further comprising labeling said mixed population of cells with said fluorescent dopamine analog following step (c) and prior to step (d).

## Patentansprüche

1. In-vitro-Verfahren zum Bestimmen, ob ein Mittel ein Neuroeffektor ist, wobei das Verfahren umfasst:
(a) Markieren von dopaminergen Neuronen, die in einer gemischten Population von Zellen mit einem fluoreszierenden Dopaminanalogon in Gegenwart eines Dopaminrezeptorantagonisten enthalten sind, wobei der Dopaminrezeptorantagonist Sulpirid oder eine Zugabe des Dopamintransporterblockers GBR umfasst;
(b) Messen eines Fluoreszenzniveaus in der gemischten Population von Zellen;
(c) Aussetzen der gemischten Population von Zellen gegenüber dem Mittel;
(d) Neuvermessen eines Fluoreszenzniveaus in der gemischten Population von Zellen, wobei eine Änderung des Fluoreszenzniveaus darauf hinweist, dass die Substanz ein Neuroeffektor ist,
wobei das fluoreszierende Dopaminanalogon ein mit Dansylmolekül markiertes Dopamin umfasst.

2. Verfahren nach Anspruch 1, wobei die gemischte Population von Zellen ferner Zellen umfasst, die einen Dopaminrezeptor exprimieren und keinen Dopamintransporter exprimieren.

3. Verfahren nach Anspruch 1, wobei die dopaminergen Neuronen durch ex vivo Differenzierung pluripotenter Stammzellen erzeugt werden.

4. Verfahren nach Anspruch 1, ferner umfassend das Markieren der gemischten Population von Zellen mit dem fluoreszierenden Dopaminanalogon nach Schritt (c) und vor Schritt (d).

## Revendications

1. Procédé in vitro pour déterminer si un agent est un neuroeffecteur, le procédé comprenant :
(a) marquer de neurones dopaminergiques qui sont compris dans une population mixte de cellules avec un analogue de dopamine fluorescent en présence d'un antagoniste de récepteur de dopamine, dans lequel ledit antagoniste de récepteur de dopamine comprend Sulpiride ou une addition du transporteur de dopamine GBR de blocage ;
(b) mesurer un niveau de fluorescence dans ladite population mixte de cellules ;
(c) exposer ladite population mixte de cellules audit agent ;
(d) remesurer un niveau de fluorescence dans ladite population mixte de cellules, dans lequel un changement dans ledit niveau de fluorescence indique que la substance est un neuroeffecteur,
dans laquelle ledit analogue de dopamine fluorescent comprend une dopamine marquée avec une molécule de dansyle.

2. Procédé selon la revendication 1, dans lequel ladite population mixte de cellules comprend en outre des cellules qui expriment un récepteur de dopamine et n'expriment pas un transporteur de dopamine.

3. Procédé selon la revendication 1, dans lequel lesdits neurones dopaminergiques sont générés par des cellules souches pluripotentes différenciantes ex vivo.

4. Procédé selon la revendication 1, comprenant en outre le marquage de ladite population mixte de cellules avec ledit analogue de dopamine fluorescent après l'étape (c) et avant l'étape (d).
